(19) Europäisches Patentamt
European Patent Office
Office européen des brevets

(11) Veröffentlichungsnummer: **0 011 712 B1**

# (12) EUROPÄISCHE PATENTSCHRIFT

(45) Veröffentlichungstag der Patentschrift: **15.06.83**

(51) Int. Cl.³: **C 25 B 3/02**, C 07 C 41/50, C 07 C 43/315, C 07 C 125/067, C 07 C 125/04

(21) Anmeldenummer: **79104147.8**

(22) Anmeldetag: **26.10.79**

(54) **Verfahren zur Herstellung von in 4-Stellung substituierten Benzaldehyddialkylacetalen.**

(30) Priorität: **08.11.78 DE 2848397**

(43) Veröffentlichungstag der Anmeldung: **11.06.80 Patentblatt 80/12**

(45) Bekanntmachung des Hinweises auf die Patenterteilung: **15.06.83 Patentblatt 83/24**

(84) Benannte Vertragsstaaten: **DE FR GB IT**

(56) Entgegenhaltungen:
**GB-A-1 230 347**
**US-A-3 607 941**
**US-A-3 933 858**
**JOURNAL OF PHARMACEUTICAL SCIENCES, Band 61, Nr. 4, April 1972, Washington, DC, US; F. GUALTIERI et al., »Topical mosquito repellents IV: Alicyclic bicyclic and unsaturated acetals aminoacetals and carboxamide acetals«, Seiten 577–580.**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Degner, Dieter, Dr., Kurpfalzstrasse 8, D-6701 Dannstadt-Schauernheim (DE)**
Erfinder: **Barl, Manfred, Dr., Merowingerstrasse 2, D-6701 Otterstadt (DE)**
Erfinder: **Siegel, Hardo, Dr., Hans-Purrmann-Allee 25, D-6720 Speyer (DE)**

EP 0 011 712 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von in 4-Stellung substituierten Benzaldehyddialkylacetalen

Diese Erfindung betrifft ein neues elektrochemisches Verfahren zur Herstellung von in 4-Stellung substituierten Benzaldehyddialkylacetalen.

Nach dem erfindungsgemäßen Verfahren werden die Benzaldehyddialkylacetale der Formel

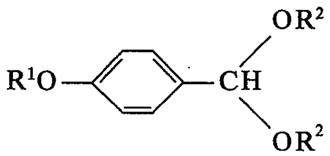

(I)

in der $R^1$ einen der Reste

$CH_2{=}CH{-}CH_2{-}$ 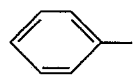

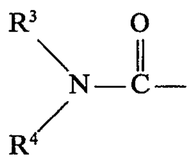 oder 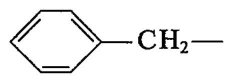

$R^2$ einen Alkylrest mit 1 bis 4 C-Atomen und die Reste $R^3$ und $R^4$ Wasserstoffatome oder Alkylgruppen mit 1 bis 6 C-Atomen bedeuten, dadurch hergestellt, daß man in 4-Stellung substituierte Methylbenzole der allgemeinen Formel

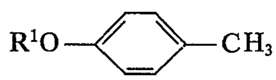

(II)

in der $R^1$ die oben genannte Bedeutung hat, in Gegenwart eines Alkohols der Formel

$$R^2OH \qquad \text{(III)}$$

in der $R^2$ die obengenannte Bedeutung hat und eines Leitsalzes, elektrochemisch oxidiert.

Ausgangsverbindungen der Formel II sind p-Benzyloxytoluol, p-Phenoxytoluol, 4-Methylphenyl-N,N-dimethylcarbamat und p-Allyloxytoluol. Als Alkohole der Formel III sind Methanol und Äthanol bevorzugt.

Es ist zwar aus J. C. S. Perkin I, 1978, Seiten 708–715, bekannt, daß man p-Methoxytoluol bzw. p-Xylol elektrochemisch zu Anisaldehyddimethylacetal bzw. 4-Methylbenzylaldehyddimethylacetal oxidieren kann. Daß sich die Ausgangsstoffe des erfindungsgemäßen Verfahrens so glatt zu dem entsprechenden Benzaldehyddialkylacetalen elektrochemisch oxidieren lassen, war jedoch überraschend, da bei der Struktur der Reste $R^1$ ein anderer Reaktionsverlauf erwartet werden mußte.

Das erfindungsgemäße Verfahren kann sowohl in einer geteilten als auch in einer ungeteilten Zelle durchgeführt werden. Als Elektrolyt dient dabei eine Lösung des substituierten Methylbenzols in dem verwendeten Alkohol, die ein Leitsalz enthält. Als Leitsalze verwendet man z. B. die in der Elektrochemie üblichen Leitsalze. Gut geeignet sind z. B. Salze, die in der zu elektrolysierenden Lösung löslich und unter den Versuchsbedingungen weitgehend stabil sind. Beispiele besonders geeigneter Leitsalze sind Fluoride, wie KF, Tetrafluorborate, wie $Et_4NBF_4$, Perchlorate, wie $Et_4NClO_4$, Sulfate, wie $Et_4NSO_4Et$, Alkoholate, wie $NaOCH_3$ und Hydroxide, wie KOH.

Die Zusammensetzungen des Elektrolyten kann bei dem erfindungsgemäßen Verfahren in weiten Grenzen gewählt werden. Die verwendeten Elektrolytlösungen haben beispielsweise folgende Zusammensetzung:

| | |
|---|---|
| 5–50 Gew.-% | Methylbenzol der Formel II |
| 50–95 Gew.-% | Alkohol der Formel III |
| 0,5–15 Gew.-% | Leitsalz |

Zur Verbeserung der Löslichkeit der Methylbenzole können gegebenenfalls zusätzlich Kolösungsmittel verwendet werden, die unter den Versuchsbedingungen weitgehend stabil sind. Beispiele hierfür sind Nitrile, wie Acetonitril, Halogenkohlenwasserstoffe, wie Methylenchlorid, Äther wie Dimethoxyäthan und Ketone wie Aceton.

Als Anodenmaterialien lassen sich bei dem erfindungsgemäßen Verfahren alle Elektrodenmaterialien einsetzen, die unter den Versuchsbedingungen beständig sind. Beispiele für geeignete

Anodenmaterialien sind Graphit, graphitgefüllte Kunststoffe, Edelmetalle, wie Platin und Gold sowie edelmetallbeschichtete Titanelektroden. Als Kathoden werden beispielsweise Graphit-, Eisen-, Stahl-, Blei- oder Edelmetallelektroden eingesetzt. Stromdichte und Umsatz können in weiten Grenzen gewählt werden. Die Stromdichten können beispielsweise 1 – 20 A/dm$^2$ betragen, die Elektrolyse selbst wird z. B. mit 2 – 15 Faraday pro Mol Ausgangsverbindung durchgeführt. Man führt die Elektrolyse z. B. bei Temperaturen zwischen 0 und 60° C durch.

Die Elektrolyseausträge werden in der Regel destillativ aufgearbeitet. Überschüssiger Alkohol und evtl. noch vorhandenes Ausgangsmaterial werden von den Acetalen durch Destillation abgetrennt und können zur Elektrolyse zurückgeführt werden. Die substituierten Benzaldehyddialkylacetale können dann beispielsweise durch Rektifikation weiter gereinigt werden. Das verwendete Leitsalz kann beispielsweise durch Filtration vor der Reindestillation der Acetale von den Acetalen abgetrennt und zur Elektrolyse zurückgeführt werden.

Die substituierten Benzaldehydacetale sowie die hieraus nach bekannten Verfahren herstellbaren Benzaldehyde sind Vorprodukte für Wirkstoffe, Riech- und Aromastoffe. 4-Benzyloxy-benzaldehyddimethylacetal läßt sich durch Umsetzung mit Säuren glatt in 4-Hydroxybenzaldehyd überführen. 4-Hydroxybenzaldehyd ist ein Vorprodukt für die Herstellung von 4-Hydroxyphenylglycin, das für die Synthese halbsynthetischer Antibiotica, wie Amoxillin, eingesetzt wird.

## Beispiel 1

### Herstellung von 4-Benzyloxy-benzaldehyddimethylacetal

| | |
|---|---|
| Apparatur | ungeteilte Zelle mit 7 Elektroden; Elektrodenabstand: 0,5 mm |
| Anoden | Graphit |
| Elektrolyt | 792 g 4-Methylphenyl-benzyläther<br>25 g KF<br>2137 g Methanol |
| Kathoden | Graphit |
| Temperatur | 35 bis 40° C |
| Stromdichte | 4,7 A/dm$^2$ |
| Elektrolyse | mit 5 F/Mol 4-Methylphenyl-benzyläther |

Der Elektrolyt wird während der Elektrolyse über einen Wärmeaustauscher gepumpt.

### Aufarbeitung

Nach Beendigung der Elektrolyse wird Methanol abdestilliert, KF (22 g) abfiltriert und der Rückstand bei 1 bis 2 Torr und 160 bis 180° C fraktioniert destilliert. Hierbei erhält man neben 27,8 g unumgesetztem 4-Methylphenyl-benzyläther 617,9 g 4-Benzyloxy-benzaldehyd-dimethylacetal. Dies entspricht einer 62,1%igen Material- und 47,9%igen Stromausbeute.

## Beispiel 2

### Herstellung von 4-Allyloxy-benzaldehyddimethylacetal

| | |
|---|---|
| Apparatur | ungeteilte Zelle mit 7 Elektroden, Elektrodenabstand: 0,5 mm |
| Anoden | Graphit |
| Elektrolyt | 296 g 4-Methylphenyl-allyläther<br>25 g KF<br>2375 g Methanol |
| Kathoden | Graphit |
| Temperatur | 25 bis 28° C |
| Stromdichte | 5,4 A/dm$^2$ |
| Elektrolyse | mit 13,4 F/Mol 4-Methylphenyl-allyläther |

Der Elektrolyt wird während der Elektrolyse über einen Wärmeaustauscher umgepumpt.

Aufarbeitung

Nach Beendigung der Elektrolyse wird Methanol abdestilliert, KF (22 g) abfiltriert und der Rückstand bei 1 bis 2 Torr und 130 bis 140°C fraktioniert destilliert. Hierbei erhält man 151 g 4-Allyloxy-benzaldehyd-dimethylacetal. Dies entspricht einer Materialausbeute von 36,3% und einer Stromausbeute von 10,8%.

Beispiel 3

Herstellung von 4-Phenoxy-benzaldehyddimethylacetal

| | |
|---|---|
| Apparatur | ungeteilte Zelle mit 7 Elektroden,<br>Elektrodenabstand: 0,5 mm |
| Anoden | Graphit |
| Elektrolyt | 100 g 4-Methylphenyl-phenyläther<br>25 g KF<br>2375 g Methanol |
| Kathoden | Graphit |
| Temperatur | 24 bis 30°C |
| Stromdichte | 5,4 A/dm$^2$ |
| Elektrolyse | mit 11 F/Mol 4-Methylphenyl-phenyläther |

Der Elektrolyt wird während der Elektrolyse über einen Wärmeaustauscher umgepumpt.

Aufarbeitung

Nach Beendigung der Elektrolyse wird Methanol abdestilliert, KF (23 g) abfiltriert und der Rückstand bei 0,5 bis 1 Torr und 110 bis 150°C fraktioniert destilliert. Hierbei erhält man 52 g 4-Phenoxy-benzaldehyddimethylacetal. Dies entspricht einer Materialausbeute von 39,2% und einer Stromausbeute von 14,3%.

Beispiel 4

Herstellung von [(4-Dimethoxymethyl)-phenyl]-N,N-dimethylcarbamat

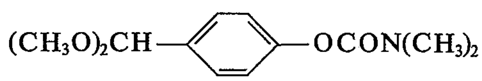

| | |
|---|---|
| Apparatur | ungeteilte Zelle mit 7 Elektroden,<br>Elektrodenabstand: 0,5 mm |
| Anoden | Graphit |
| Elektrolyt | 330 g p-Kresyl-N,N-dimethylcarbamat<br>2370 g Methanol<br>25 g KF |
| Kathoden | Graphit |
| Temperatur | 25 bis 30°C |
| Stromdichte | 4,7 A/dm$^2$ |
| Elektrolyse | mit 10 F/Mol p-Kresyl-N,N-dimethylcarbamat |

Der Elektrolyt wird während der Elektrolyse über einen Wärmeaustauscher umgepumpt.

Aufarbeitung

Nach Beendigung der Elektrolyse wird Methanol abdestilliert, KF (23 g) abfiltriert und der Rückstand bei 60 bis 170°C und 2 Torr fraktioniert destilliert. Hierbei erhält man 97 g p-Kresol, 52 g unumgesetztes p-Kresyl-N,N-dimethylcarbamat und 150 g [(4-Dimethoxymethyl)-phenyl]-N,N-dimethylcarbamat. Dies entspricht einer Materialausbeute von 40,4%.

**Patentanspruch**

Verfahren zur Herstellung von in 4-Stellung substituierten Benzaldehyddialkylacetalen der Formel

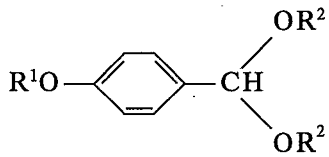
(I)

in der $R^1$ einen der Reste

$CH_2{=}CH{-}CH_2{-}$ 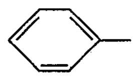

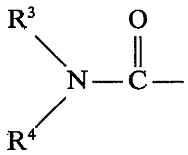 oder 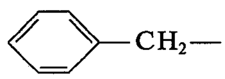

$R^2$ einen Alkylrest mit 1 bis 4 C-Atomen und die Reste $R^3$ und $R^4$ Wasserstoffatome oder Alkylgruppen mit 1 bis 6 C-Atomen bedeuten, dadurch gekennzeichnet, daß man in 4-Stellung substituierte Methylbenzole der allgemeinen Formel

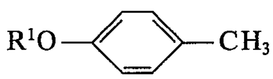
(II)

in der $R^1$ die oben genannte Bedeutung hat, in Gegenwart eines Alkohols der Formel

$R^2OH$ (III)

in der $R^2$ die oben genannte Bedeutung hat, und eines Leitesalzes, elektrochemisch oxidiert.

**Claim**

A process for the preparation of a 4-substituted benzaldehydedialkylacetal of the formula

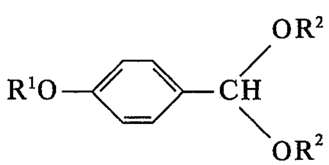
(I)

where $R^1$ is one of the radicals

$CH_2{=}CH{-}CH_2{-}$ 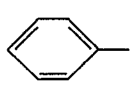

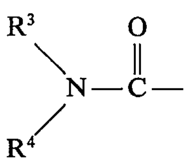 and 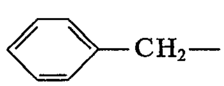

$R^2$ is alkyl of 1 to 4 carbon atoms and $R^3$ and $R^4$ are hydrogen or alkyl of 1 to 6 carbon atoms, wherein a 4-substituted methylbenzene of the general formula

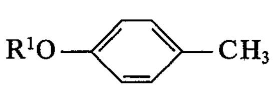
(II)

where $R^1$ has the meaning given in claim 1, is electrochemically oxidized in the presence of an alcohol of the formula

$R^2OH$ (III)

where $R^2$ has the above meaning, and of a conductive salt.

**Revendication**

Procédé pour la préparation de benzaldéhyde-dialcoylacétals substitués en position 4 de formule

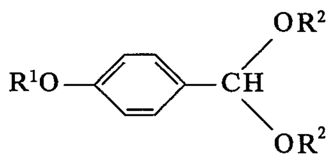

(I)

dans laquelle $R^1$ représente l'un des radicaux

$CH_2{=}CH{-}CH_2{-}$ 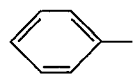

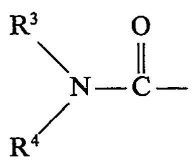 ou 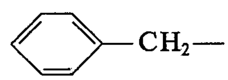

$R^2$ un radical alcoyle à 1−4 atomes de C et les radicaux $R^3$ et $R^4$ sont des atomes d'hydrogène ou des groupes alcoyles à a−6 atomes de C, caractérisé en ce qu'on oxyde électrochimiquement des méthylbenzènes substitués en position 4 de formule générale

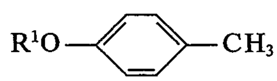

(II)

dans laquelle $R^1$ a la signification donnée ci-cessus, en présence d'un alcool de formule

$R^2OH$ (III)

dans laquelle $R^2$ a la signification donnée ci-dessus et en présence d'un sel conducteur.